Europäisches Patentamt

European Patent Office

Office européen des brevets

(19)

(11) Veröffentlichungsnummer: **0 362 382**

**A1**

(12) **EUROPÄISCHE PATENTANMELDUNG**
veröffentlicht nach Art. 158 Abs. 3
EPÜ

(21) Anmeldenummer: 88905467.2

(22) Anmeldetag: 17.03.88

(86) Internationale Anmeldenummer:
PCT/SU88/00060

(87) Internationale Veröffentlichungsnummer:
WO 89/08399 (21.09.89 89/23)

(51) Int. Cl.5: **A01N 41/12 , A01N 41/10 ,
A01N 37/36**

(43) Veröffentlichungstag der Anmeldung:
11.04.90 Patentblatt 90/15

(84) Benannte Vertragsstaaten:
**CH DE FR GB IT LI**

(71) Anmelder: **VSESOJUZNY
NAUCHNO-ISSLEDOVATELSKY INSTITUT
SELSKOKHOZAISTVENNOI BIOTEKHNOLOGII
VSESOJUZNOI AKADEMII
SELSKOKHOZYAISTVENNYKH
NAUK IMENI V.I. LENINA
ul. Pskovskaya 12-4 Moscow, 127253(SU)**

Anmelder: **VSESOJUZNY
NAUCHNO-ISSLEDOVATELSKY INSTITUT
RASTENIEVODSTA IMENI N.I. VAVILOVA
VSESOJUZNOI AKADEMII
SELKOKHOZYAISTVENNYKH
NAUK IMENI V.I. LENINA
ul. gertsena, 44 Leningrad, 190000(SU)**

Anmelder: **INSTITUT
ELEMENTOORGANICHESKIKH SOEDINENY
IMENI A.N. NESMEY ANOVA AKADEMII NAUK
SSSR
ul. Vavilova, 28
Moscow, 117813(SU)**

(72) Erfinder: **FEDIN, Marat Alexandrovich**
ul. Dm.Ulyanova, 24-158
Moscow, 107078(SU)
Erfinder: **KUZNETSOVA, Tatyana
Alexandrovna**
Khoroshevskoe shosse, 36b-62
Moscow, 123007(SU)
Erfinder: **SAVCHUK, Valentin A.**

Opytnaya stantsia VIR Globinsky raion
Poltavskaya obl. selo Ustimovka, 315967(SU)
Erfinder: **NOVIKOVA, Svetlana Alexandrovna**
ul. Shirokaya, 19-2-176
Moscow, 129224(SU)
Erfinder: **AMINOV, Sabir Nigmatovich**
ul. Mushinova, 4/1-12
Tashkent, 700041(SU)
Erfinder: **PETROVA, Raisa Grigorievna**
ul. Volgina, 15-1-63
Moscow, 117425(SU)
Erfinder: **CHURKINA, Tatyana Dmitrievna**
ul. Ulbrikhta, 24-6
Moscow, 125252(SU)
Erfinder: **KOGAN, Alexandr Semenovich**
Varshavskoe shosse, 144-2-367
Moscow, 113519(SU)
Erfinder: **EGOROV, Boris Mikhailovich**
ul. Chkalova, 48a-51
Moscow, 107020(SU)
Erfinder: **POVOLOTSKY, Dmitry Ivanovich**
1 Khoroshevsky proezd, 14-1-16
Moscow, 125284(SU)
Erfinder: **ALSING, Tamara Karlovna**
Zagrebsky bulvar, 7-1-455
Leningrad, 192284(SU)
Erfinder: **VOSKOBOINIK, Leonid
Konstantinovich**
ul. Pushkina, 35-6
Krasnodar, 350023(SU)
Erfinder: **FEDORENKO, Tatyana Sergeevna**
ul. Shkolnaya, 11-23
Krasnodar, 350059(SU)
Erfinder: **FREIDLINA, Rakhil Khatskelevna**
(deceased)

(SU)

EP 0 362 382 A1

Erfinder: **GYSKA, Mikhail Nikolaevich (deceased)**

(SU)

(74) Vertreter: **von Füner, Alexander, Dr. et al**

**Patentanwälte v. Füner, Ebbinghaus, Finck**

**Mariahilfplatz 2 & 3**

**D-8000 München 90(DE)**

(54) **VERFAHREN ZUR STERILISATION VON PFLANZLICHEN POLLEN.**

(57) A method of sterilizing plant pollen comprises treating the plants with a sterilizing agent together with a diluent during the fifth and/or the sixth stage of organogenesis. The sterilizing agents used are thiocarbonic acids and/or their derivatives of the general formula: RXR', where $X = S, SO_2$; $R = (CH_2)_4CO_2H$, $(CH_2)_4CO_2Na$, $(CH_2)_4CO_2K$, $(CH_2)_4CO_2C_4H_9$, $(CH_2)_4CO_2C_8H_{17}$, $C_{10}H_{21}$; $R' = (CH_2)_4CO_2H$, $(CH_2)_4CO_2Na$, $(CH_2)_4CO_2K$, $(CH_2)_4CO_2C_4H_9$, $(CH_2)_4CO_2C_8H_{17}$.

EPAA-38372.2

# VERFAHREN ZUR STERILISATION DER STAUBBEUTEL VON PFLANZEN

## Gebiet der Technik

Die vorliegende Erfindung bezieht sich auf die Biologie und Landwirtschaft und betrifft insbesondere ein Verfahren zur Sterilisation der Staubbeutel von Pflanzen, welches in der Selektion und im Samenbau zur Verwendung kommt.

## Zugrundeliegender Stand der Technik

Heutzutage wird in der Welt die Aufgabe gelöst, die Landwirtschaft zu intensivieren, insbesondere die Ertragsfähigkeit von Getreide-, Futter-, Gemüse- und technischen Kulturen durch eine breite Verwendung von Hybriden der ersten Generation zu steigern. Wegen ihrer Hybridkraft unterscheiden sich die Hybride von den Elternformen durch eine höhere Leistung (um 25 bis 30 %) und eine bessere Produktqualität. Es besteht ein Verfahren zur Erzeugung von neuen Hybriden, welches auf einem System "zytoplasmatische Pollensterilität – Restorer der Fertilität" beruht. Diesem Verfahren liegt eine mehrere Jahre lang (12 bis 14 Jahre) dauernde und komplizierte Selektionsarbeit zugrunde, die das Schaffen von sterilen Analoga, Fixatoren der Sterilität und Restorern der Fertilität umfaßt. Besonders aussichstreich sind Verfahren, die auf der Sterilisation der Staubbeutel von Pflanzen mit chemischen Sterilisationsmitteln (Gametoziden) basieren. Die Verwendung von Gametoziden ist bedeutend wirtschaftlicher als die des Systems "zytoplasmatische Pollensterilität", weil die Notwendigkeit entfällt, solche Formen wie steriles Analogon, Analoga für die Fixierung der Sterilität bei mütterlichen Formen und für die Restauration der Fertilität bei väterlichen Formen zu erzeugen. Man kann praktisch die Samen von Hybriden der ersten Generation sowohl im Laufe der Selektionsforschung von Ausgangsformen, als auch bei der Organisation ihrer technischen Herstellung gewinnen.

Zur Zeit sind etwa 200 Verbindungen gefunden, welche eine gametozide Aktivität besitzen und ihrer chemischen Struktur nach zu verschiedenen Klassen von chemischen Verbindungen gehören. Gametozide müssen die volle Pollensterilität bei behandelten Pflanzen unter Erhaltung der Lebensfähigkeit von Eizellen bewirken und eine ausreichend hohe Fähigkeit (mindenstens 70 % der Kontrolle), den Fruchtknoten unter freier Bestäubung zu bilden, sicherstellen. Die Werte ihrer Phytotoxizität und Toxizität für Warmblüter müssen minimal sein.

Es sind Verfahren zur Sterilisation der Staubbeutel von Getreidekulturen (L.Dzh. Nikell, "Regulatory rosta rasteny. Primenenie v selskom khozyaistve", Moskva, izdatelstvo "Kolos", 1984, S. 28 bis 31; SU, A, 906457; deutsch: L.J. Nikelll, "Wachstumsregulatoren von Pflanzen, Anwendung in der Landwirtschaft", Moskau, Verlag "Kolos", 1984. S. 28 bis 31) bekannt, welche in der Behandlung von Pflanzen mit Sterilisationsmitteln wie 2-Chloräthylphosphonsäure (Ethrel), Maleinsäurehydrazid, Di-(polyfluoralkyl)--phosphorsäuren und ihre Salze u.a. bestehen. Die Behandlung der Pflanzen mit heimischen Sterilisationsmitteln erfolgt in der V. und/oder VI. Periode der Organogenese (nach F.M. Kupermann).

In der V. Periode der Organogenese setzen die Prozesse der Bildung und der Differenzierung von Blüten ein. Gegen Ende dieser Periode entstehen Neubildungen - sporogene Archensporgewebe. Während dieser Periode kommt es zur Anlegung von Staubblättern, Stempel und Blütenhülle. In der V. Periode tritt die Differenzierung des Höckers von Staubblättern in Staubfaden und Stempel in Erscheinung. Die VI. Periode ist durch Ablauf der Prozesse der Blütenbildung (Mikro- und Makrosporogenese) gekennzeichnet. In dieser Periode werden gesonderte einkernige Pollenkörner gebildet (F.M.Kupermann, "Morfofiziologia rasteny". Moskva, izdatelstvo "Vysshaya shkola", 1973, S. 30 bis 36; deutsch "Morphophysiologie von Pflanzen", Moskau, Verlag "Wysschaja schkola", 1973, S. 30 bis 36).

Es ist weiter ein Verfahren zur Sterilisation der Staubbeutel von Graspflanzen (GB, A, 1567153) bekannt, das darin besteht, daß man die Behandlung von Graspflanzen mit einem Sterilisationsmittel in der Periode durchführt, die zwischen Auftreten des zweiten Stengelglieds und Ährenschieben liegt. Als Sterilisationsmittel benutzt man heterozyklische Verbindungen, deren Hauptvertreter 2-Karboxy-3,4--methanpyrrolidin oder 2-Methoxykarbonyl-3,4-methanpyrrolidin sind. Die angegebenen Verbindungen werden in Kombination mit Verdünnungsmitteln und oberflächenaktiven Stoffen eingesetzt.

Offenbarung der Erfindung

Der Erfindung liegt die Aufgabe zugrunde, durch die Auswahl von Sterilisationsmitteln ein Verfahren zu entwickeln, das zur Sterilisation der Staubbeutel eines breiten Spektrums von Kulturen mit hohem Sterilisationsgrad unter Erhaltung der hohen Fähigkeit von Samen, Fruchtansätze bei freier Bestäubung zu bilden, verwendet werden kann.

Die Aufgabe wird dadurch gelöst, daß in einem Verfahren zur Sterilisation der Staubbeutel von Pflanzen durch ihre Behandlung mit einem Sterilisationsmittel in Verbindung mit einem Verdünner in der fünften und/oder sechsten Periode der Organogenese erfindungsgemäß als Sterilisationsmittel Thiokarbonsäuren und/oder ihre Derivate der allgemeinen Formel RXR' dienen, worin X für $S$, $SO_2$, R für $(CH_2)_4CO_2H$, $(CH_2)_4CO_2Na$, $(CH_2)_4CO_2K$, $(CH_2)_4CO_2C_4H_9$, $(CH_2)_4CO_2C_8H_{17}$, $C_{10}H_{21}$, R' für $(CH_2)_4CO_2H$, $(CH_2)_4CO_2Na$, $(CH_2)_4CO_2K$, $(CH_2)_4CO_2C_4H_9$, $(CH_2)_4CO_2C_8H_{17}$ stehen.

Das Sterilisationsmittel kann in Verbindung mit einem beliebigen bekannten und dazu geeigneten Verdünner verwendet werden. Zweckmäßigerweise wird es in Verbindung mit Wasser als 0,1- bis 2%ige wäßrige Emulsion benutzt. Als Pflanzen, die mit dem angegebenen Sterilisationsmittel behandelt werden, dienen bevorzugt Graspflanzen oder Sonnenblume.

Das erfindungsgemäße Verfahren ermöglicht es, die männliche Sterilität von Pflanzen (98 bis 100 %) zu erzielen und einen hohen Prozentsatz (über 70 %) der Fähigkeit zur Samenbildung zu erhalten. Zwecks Erzielung eines hohen Sterilisationsgrades von Staubbeuteln unter ungünstigen Klimabedingungen wird die Behandlung von Pflanzen mit dem Sterilisationsmittel in der fünften und/oder sechsten Periode der Organogenese (nach Kupermann) wiederholt.

Beste Durchführungsvariante der Erfindung

Das erfindungsgemäße Verfahren wird wie folgt durchgeführt.

Pflanzen wie, z.B., Winter- und Sommerweizen, diploider und tetraploider Roggen, Tritikale, Hirse, Sonnenblume, Graspflanzen werden mit einem Sterilisationsmittel behandelt, wobei als solches die erwähnten Thiokarbonsäuren und/oder ihre Derivate verwendet werden.

Thiokarbonsäuren oder ihre Derivate können in Verbindung mit beliebigen geeigneten Verdünnern verwendet werden, als Verdünner wird Wasser zweckmäßigerweise benutzt. Dabei wird eine 0,1- bis 2%ige wäßrige Emulsion der angegebenen Verbindungen vorzugsweise verwendet.

Man kann den gebrauchsfertigen Lösungen beliebige geeignete oberflächenaktive Stoffe wunschgemäß zusetzen. Beim Aufbringen auf die Pflanzen sind gewöhnlich den gebrauchsfertigen Lösungen beliebige bekannte Hilfszusätze wie Netzmittel, Dispergiermittel und Adhäsionsmittel hinzuzufügen.

Das Sterilisationsmittel läßt sich auf Pflanzen nach verschiedenen Behandlungsverfahren wie Flüssigkeitszerstäubung und Luftzerstäubung (Aerosole) aufbringen. Die Behandlung von Pflanzen mit dem Sterilisationsmittel erfolgt in der fünften und/oder sechsten Periode der Organogenese (nach Kupermann). Die Aufwandmenge des Sterilisationsmittels hängt von der Natur der Verbindung, von der zu behandelnden Kultur, der Behandlungsperiode und natürlichen Klimafaktoren ab. Um einen hohen Sterilisationseffekt unter ungünstigen Klimabedingungen sicherzustellen, ist es zweckmäßig, die wiederholte Behandlung von Pflanzen in der VI. Periode der Organogenese durchzuführen. Die Gesamtdosis des Sterilisationsmittels beträgt 0,6 bis 20 kg/ha.

Alle erfindungsgemäßen Thiokarbonsäuren oder ihre Derivate, die als Sterilisationsmittel zur Verwendung kommen, waren auf die Toxizität im Tierversuch geprüft. Die Prüfergebnisse haben gezeigt, daß die angegebenen Verbindungen von schwacher oder mittlerer Toxizität sind. So beträgt beispielsweise $LD_{50}$ mehr als 2000 mg/kg für Ratten bei Bis (2-äthylhexyläther) der $\delta$, $\delta'$-Thiodivaleriansäure, bei anderen Verbindungen liegen die $LD_{50}$ -Werte auf dem gleichen Niveau.

Die gametozide Aktivität der erfindungsgemäßen Sterilisationsmittel war im Feldversuch in verschiedenen bodenklimatischen Zonen mit Teilstücken von 10 $m^2$ Größe in drei- bis viermaliger Wiederholung festgestellt. Jedes Sterilisationsmittel wurde mindenstens 5 Jahre lang geprüft.

Die Entwicklung der Perioden der Organogenese wird zytologisch überwacht. Die Behandlung von Pflanzen mit dem Sterilisationsmittel erfolgt am Anfang der fünften Periode der Organogenese nach Kupermann.

Mit dem Ährenschieben verwirklicht man die Isolierung von Hauptähren und anderen Stockwerken in Pergamentbeuteln. Bei Weizen und Tritikale werden Einzelisolatoren benutzt. Bei Roggen schließt man je Ähre 5 bis 7 verschiedene nebeneinander befindliche Pflanzen unter einen gemeinsamen Isolator ein. Bei Hierse wird jede Rispe getrennt isoliert. Der Sterilitätsprozentsatz (X) für Weizen, Roggen, Tritikale und Hirse wird nach der Formel

$$X = \left[ 1 - \frac{\text{Anzahl der in einem Isolator befruchteten Samen von behandelten Pflanzen}}{\text{Anzahl der in einem Isolator befruchteten Samen von unbehandelten Kontrollpflanzen}} \right] \times 100\ \%$$

ermittelt.

Die Kornzahl in nicht isolierten Ähren von Kontrollpflanzen wird bedingt für 100 % Ansetzen bei freier Bestäubung angenommen.

Um zuverlässige Angaben zu erhalten, verwendet man 20 bis 25 Isolatoren jeder Wiederholung für Weizen und Tritikale, 10 bis 15 Isolatoren jeder Wiederholung für Roggen und Hirse.

Zur Kontrolle der chemischen Sterilisation des Sonnenblumenpollens werden für jede Verbindung 45 behandelte Pflanzen jeder Wiederholung benutzt, wobei 15 Pflanzen von denen zwecks Selbsbestäubung isoliert werden, Blütenkörbchen anderer 15 Pflanzen mit einem Pollengemisch, gesammelt von 20 bis 25 behandelten isolierten Körbchen, bestäubt werden, und 15 Pflanzen für freie Bestäubung gelassen werden, damit das Ansetzen von Achänen mit dem Pollen der väterlichen Form kontrolliert werden kann.

Man beurteilt die Pollensterilität von Pflanzen der Sonnenblume nach der Pollenfertilität und -keimfähigkeit, nach den morphologischen Besonderheiten von Spermien und nach der Fähigkeit von Achänen zum Ansetzen bei der Bestäubung der behandelten isolierten Pflanzen mit dem Pollen der unbehandelten väterlichen Form. Die Lebensfähigkeit der Eizelle wird nach der Samenbildung von behandelten Pflanzen bei freier Bestäubung mit der väterlichen Form bestimmt.

Es ist wünschenswert, die Behandlung von Pflanzen bei heiterem windstillem Wetter durchzuführen. Alle Verbindungen dringen in Gewebe der Pflanzen innerhalb von 4 Stunden nach der Behandlung völlig durch. Im Falle der Niederschläge innerhalb dieser 4 Stunden ist es notwendig, Pflanzen in der VI. Periode der Organogenese wiederholt zu behandeln.

Thiokarbonsäuren und ihre Derivate werden nach den bekannten Verfahren hergestellt. Was Bis(2-äthylhexyläther) der $\delta$, $\delta'$-Thiodivaleriansäure anbetrifft, so ist es nach dem Analogieverfahren zur Herstellung von Bis(di-n-butyläther) $\delta$, $\delta'$-Thiodivaleriansäure erhalten.

Zum besseren Verstehen der vorliegenden Erfindung werden folgende Durchführungsbeispiele des erfindungsgemäßen Verfahrens angeführt.

Beispiel 1

Pflanzen des Winterweizens Sorte Mironowskaja 808 werden in der V. Periode der Organogenese durch Zerstäubung der 2%igen wäßrigen Emulsion der $\delta$, $\delta'$-Thiodivaleriansäure mit Hilfe einer Rückenspritze behandelt. Als Emulgiermittel wird 0,1 Masse% Kalziumalkylbenzolsulfonat mit 12 bis 14 Kohlenstoffatomen hinzugefügt. Als Adjuvans führt man in die Emulsion 0,01 Masse% Dimethylsulfoxid ein. Der Verbrauch an Präparat beträgt 20 kg/ha. Als Kontrolle dienen Pflanzen, behandelt mit einem Verdünner ohne Sterilisationsmittel. Die Ergebnisse sind in der Tabelle 1 angegeben. Die ähnlichen Ergebnisse sind bei der Behandlung des Winterweizens Sorte Mironowskaja 808 in der VI. Periode der Organogenese erhalten.

Beispiele 2 bis 8

Der Prozeß erfolgt analog zu dem im Beispiel 1 beschriebenen. Als Sterilisationsmittel benutzt man 2%ige wäßrige Emulsion von Natriumsalz der $\delta, \delta'$ -Thiodivaleriansäure, $\delta, \delta'$ -Sulfonyldivaleriansäure, Natriumsalz der $\delta, \delta'$ -Sulfonyldivaleriansäure, Kaliumsalz der $\delta, \delta'$ -Sulfonyldivaleriansäure, Bis(di-n-butyläther) der $\delta, \delta'$ -Thiodivaleriansäure, 4-Karboxybutyldezylsulfid bzw. Bis(2-äthylhexyläther) der $\delta, \delta'$ -Thiodivaleriansäure. Die Prüfergebnisse sind in der Tabelle 1 angeführt. Die ähnlichen Ergebnisse sind bei der Behandlung des Winterweizens Sorte Mironowskaja 808 in der VI. Periode der Organogenese erhalten.

Tabelle 1

| lfd. Nr. | Beispiel Nr. | Anzahl von Körnern der Ähre im Isolator | Prozentsatz der Sterilität | Anzahl von Körnern der Ähre bei freier Bestäubung | Prozentsatz des Ansetzens von Körnern bei freier Bestäubung |
|---|---|---|---|---|---|
| 1 | Kontrolle | 39,5 | 0,0 | 41,2 | 100,0 |
| 2 | Beispiel 1 | 0,5 | 98,8 | 37,0 | 89,8 |
| 3 | Beispiel 2 | 0,3 | 99,3 | 35,3 | 85,7 |
| 4 | Beispiel 3 | 0,0 | 100,0 | 37,2 | 90,3 |
| 5 | Beispiel 4 | 0,1 | 99,8 | 34,1 | 82,8 |
| 6 | Beispiel 5 | 0,1 | 99,8 | 40,7 | 98,8 |
| 7 | Beispiel 6 | 0,0 | 100,0 | 34,8 | 84,5 |
| 8 | Beispiel 7 | 0,0 | 100,0 | 35,6 | 86,4 |
| 9 | Beispiel 8 | 0,0 | 100,0 | 36,1 | 87,6 |

Beispiele 9 bis 11

Pflanzen des Winterweizens Sorte Korall werden in der V. Periode der Organogenese (nach Kupermann) mit 2%iger wäßriger Emulsion von Bis(2-äthylhexyläther) der $\delta, \delta'$- -Thiodivaleriansäure, 4-Karboxybutyldezylsulfid bzw. Bis(di- -n-butyläther) der $\delta, \delta'$-Thiodivaleriansäure behandelt. Die Emulsion enthält 0,1 Masse% Kalziumalkylbenzolsulfonat mit 12 bis 14 Kohlenstoffatomen und 0,01 Masse% Dodezylsulfat. Der Verbrauch an Präparat beträgt 12 kg/ha. Als Kontrolle dienen Pflanzen, behandelt mit einem Verdünner ohne Sterilisationsmittel. Die Prüfergebnisse sind in der Tabelle 2 angegeben.

Die ähnlichen Ergebnisse sind bei der Behandlung des Winterweizens Korall in der VI. Periode der Organogenese erhalten.

Tabelle 2

| lfd. Nr. | Beispiel Nr. | Anzahl von Kör- nern der Ähre im Isolator | Prozentsatz der Steri- lität | Anzahl von Kör- nern der Ähre bei freier Bestäu- bung | Prozent- satz des An- set- zens von Kör- nern bei frei- er Be- stäubung |
|---|---|---|---|---|---|
| 1 | Kontrolle | 29,6 | 0,0 | 32,4 | 100,0 |
| 2 | Beispiel 9 | 0,0 | 100,0 | 30,8 | 95,1 |
| 3 | Beispiel 10 | 0,1 | 99,5 | 29,1 | 89,8 |
| 4 | Beispiel 11 | 0,1 | 99,5 | 30,2 | 93,2 |

Beispiele 12 bis 14

Pflanzen des Winterweizens Gordeiforme 12303 werden in der V. Periode der Organogenese (nach Kupermann) mit 2%iger wäßriger Emulsion von 4-Karboxybutyldezylsulfid, Bis(2-äthylhexyläther) der $\delta, \delta'$-Thiodivaleriansäure, $\delta, \delta'$-Sulfonyldivaleriansäure behandelt. Die Emulsion enthält als Emulgiermittel 0,1 Masse% Kalziumalkylbenzolsulfonat mit 12 bis 14 Kohlenstoffatomen und 0,01 Masse% Dimethylformamid. Der Verbrauch an Präparat beträgt 16 kg/ha.

Als Kontrolle dienen Pflanzen, behandelt mit einem Verdünner ohne Sterilisationsmittel.

Die Prüfergebnisse sind in der Tabelle 3 angegeben. Die ähnlichen Ergebnisse sind bei der Behandlung des Winterweizens Gordeiforme 12303 in der VI. Periode der Organogenese erhalten.

Tabelle 3

| lfd. Nr. | Beispiel Nr. | Anzahl von Körnern der Ähre im Isolator | Prozentsatz der Sterilität | Anzahl von Körnern der Ähre bei freier Bestäubung | Prozentsatz des Ansetzens von Körnern bei freier Bestäubung |
|---|---|---|---|---|---|
| 1 | Kontrolle | 28,5 | 0,0 | 29,1 | 100,0 |
| 2 | Beispiel 12 | 0,0 | 100,0 | 21,6 | 74,2 |
| 3 | Beispiel 13 | 0,0 | 100,0 | 25,3 | 86,9 |
| 4 | Beispiel 14 | 0,1 | 99,7 | 23,6 | 81,1 |

Beispiele 15 bis 22

Pflanzen des Sommerweizens Sorte Moskowskaja 35 werden in der V. Periode der Organogenese (nach Kupermann) mit 1%iger wäßriger Emulsion von $\delta, \delta'$-Thiodivaleriansäure, Natriumsalz der $\delta, \delta'$-Thiodivaleriansäure, $\delta, \delta'$-Sulfonyldivaleriansäure, Natriumsalz der $\delta, \delta'$-Sulfonyldivaleriansäure, Kaliumsalz der $\delta, \delta'$-Sulfonyldivaleriansäure, Bis (die-n-butyläther) der $\delta, \delta'$-Thiodivaleriansäure, 4-Karboxybutyldezylsulfid, Bis(2-äthylhexyläther) der $\delta, \delta'$-Thiodivaleriansäure behandelt. Die Emulsion enthält 0,1 Masse% Kalziumalkylbenzolsulfonat mit 12 bis 14 Kohlenstoffatomen und 0,01 Masse% Dimethylsulfoxid. Der Verbrauch an Präparat beträgt 6 kg/ha. Als Kontrolle dienen Pflanzen, behandelt mit einem Verdünner ohne Sterilisationsmittel. Die Prüfergebnisse sind in der Tabelle 4 angegeben. Die ähnlichen Ergebnisse sind bei der Behandlung des Winterweizens Moskowskaja 35 in der VI. Periode der Organogenese erhalten.

Tabelle 4

| lfd. Nr. | Beispiel Nr. | Anzahl von Körnern der Ähre im Isolator | Prozentsatz der Sterilität | Anzahl von Körnern der Ähre bei freier Bestäubung | Prozentsatz des Ansetzens von Körnern bei freier Bestäubung |
|---|---|---|---|---|---|
| 1 | Kontrolle | 28,6 | 0,0 | 31,9 | 100,0 |
| 2 | Beispiel 15 | 0,9 | 97,0 | 31,7 | 98,3 |
| 3 | Beispiel 16 | 0,9 | 97,0 | 30,8 | 96,5 |
| 4 | Beispiel 17 | 0,0 | 100,0 | 26,7 | 83,6 |
| 5 | Beispiel 18 | 0,3 | 99,0 | 31,1 | 97,4 |
| 6 | Beispiel 19 | 0,3 | 99,0 | 30,5 | 95,6 |
| 7 | Beispiel 20 | 0,0 | 100,0 | 28,7 | 89,9 |
| 8 | Beispiel 21 | 0,0 | 100,0 | 26,9 | 84,3 |
| 9 | Beispiel 22 | 0,0 | 100,0 | 25,7 | 80,6 |

Beispiele 23 bis 25

Pflanzen des Sommerweizens Sorte Rodina werden in der V. Periode der Organogenese (nach Kupermann) mit 1%iger wäßriger Emulsion von $\delta$, $\delta'$-Sulfonyldivaleriansäure, 4-Karboxybutyldezylsulfid, Bis(2-äthylhexyläther) der $\delta$, $\delta'$-Thiodivaleriansäure behandelt. Die Emulsion enthält 0,1 Masse% Kalziumalkylbenzolsulfonat mit 12 bis 14 Kohlenstoffatomen und 0,01 Masse% Tetrahydrofuran. Der Verbrauch an Präparat beträgt 6 kg/ha. Als Kontrolle dienen Pflanzen, behandelt mit einem Verdünner ohne Sterilisationsmittel. Die Prüfergebnisse sind in der Tabelle 5 angegeben. Die ähnlichen Ergebnisse sind bei der Behandlung des Sommerweizens Sorte Rodina in der VI. Periode der Organogenese erhalten.

Tabelle 5

| lfd. Nr. | Beispiel Nr. | Anzahl von Körnern der Ähre im Isolator | Prozentsatz der Sterilität | Anzahl von Körnern der Ähre bei freier Bestäubung | Prozentsatz des Ansetzens von Körnern bei freier Bestaubung |
|---|---|---|---|---|---|
| 1 | Kontrolle | 35,3 | 0,0 | 34,5 | 100,0 |
| 2 | Beispiel 23 | 0,0 | 100,0 | 36,1 | 96,3 |
| 3 | Beispiel 24 | 0,0 | 100,0 | 29,8 | 79,5 |
| 4 | Beispiel 25 | 0,0 | 100,0 | 31,2 | 83,2 |

Beispiele 26 und 27

Pflanzen des Sommerwizens Sorte Charkowskaja 9 werden in der V. Periode der Organogenese (nach Kupermann) mit 1%iger waßriger Emulsion von Bis(di-n-butyläther) der $\delta$, $\delta'$-Thiodivaleriansäure, Bis(2-äthylhexyläther) der $\delta$, $\delta'$-Thiodivaleriansäure behandelt. Die Emulsion enthält 0,1 Masse% Kalziumalkylbenzolsulfonat mit 12 bis 14 Kohlenstoffatomen und 0,01 Masse% Äthylenglykol. Der Verbrauch an Präparat beträgt 8 kg/ha. Als Kontrolle dienen Pflanzen, behandelt mit einem Verdünner ohne Sterilisationsmittel. Die Prüfergebnisse sind in der Tabelle 6 angegeben. Die ähnlichen Ergebnisse sind bei der Behandlung des Sommerweizens Sorte Charkowskaja 9 in der VI. Periode der Organogenese erhalten.

Tabelle 6

| lfd. Nr. | Beispiel Nr. | Anzahl von Körnern der Ähre im Isolator | Prozentsatz der Sterilität | Anzahl von Körnern der Ähre bei freier Bestäubung | Prozentsatz des Ansetzens von Körnern bei freier Bestäubung |
|---|---|---|---|---|---|
| 1 | Kontrolle | 29,2 | 0,0 | 32,2 | 100,0 |
| 2 | Beispiel 26 | 0,0 | 100,0 | 28,7 | 89,1 |
| 3 | Beispiel 27 | 0,0 | 100,0 | 28,6 | 88,9 |

Beispiele 28 bis 31

Pflanzen des Sommerweizens Sorte Botanitscheskaja 4 werden in der V. Periode der Organogenese (nach Kupermann) mit 1%iger wäßriger Emulsion von Natriumsatz der $\delta$, $\delta'$--Thiodivaleriansäure, Bis(di-n-butyläther) der $\delta$, $\delta'$-Thio-divaleriansäure, 4-Karboxybutyldezylsulfid, Bis(2-äthyl-hexyläther) der $\delta$, $\delta'$-Thiodivaleriansäure behandelt. Die Emulsion enthält 0,1 Masse% Kalziumalkylbenzolsulfonat mit 12 bis 14 Kohlenstoffatomen und 0,01 Masse% Dodezylsulfat. Der Verbrauch an Präparat beträgt 6 kg/ha. Als Kontrolle dienen Pflanzen, behandelt mit einem Verdünner ohne Sterilisationsmittel. Die Prüfergebnisse sind in der Tabelle 7 angegeben. Die ähnlichen Ergebnisse sind bei der Behandlung des Weizens Botanitscheskaja 4 in der VI. Periode der Organogenese erhalten.

Tabelle 7

| lfd. Nr. | Beispiel Nr. | Anzahl von Kör-nern der Ähre im Isolator | Prozent-satz der Sterili-tät | Anzahl von Kör-nern der Ähre bei freier Be-stäubung | Prozent-satz des Ansetzens von Kör-nern bei freier Be-stäubung |
|---|---|---|---|---|---|
| 1 | Kontrolle | 36,9 | 0,0 | 40,2 | 100,0 |
| 2 | Beispiel 28 | 0,3 | 99,2 | 36,2 | 90,0 |
| 3 | Beispiel 29 | 0,0 | 100,0 | 30,6 | 76,2 |
| 4 | Beispiel 30 | 0,0 | 100,0 | 32,8 | 81,6 |
| 5 | Beispiel 31 | 0,0 | 100,0 | 33,4 | 83,1 |

Beispiele 32 bis 36

Pflanzen von diploidem Roggen Sorte Tschulpan werden in der V. und VI. Periode der Organogenese (nach Kupermann) mit 1%iger wäßriger Emulsion von $\delta$, $\delta'$-Sulfonyldivaleran-säure, Kaliumsatz der $\delta$, $\delta'$-Sulfonyldivaleriansäure (Bis(di-n-butyläther) der $\delta$, $\delta'$-Thiodivaleriansäure, Bis(2-äthylhexyläther) der $\delta$, $\delta'$-Thiodivaleriansäure, 4-Karboxybutyldezylsulfid behandelt. Die Emulsion enthält 0,1 Masse% Kalziumalkylbenzolsulfonat mit 12 bis 14 Kohlenstoffatomen und 0,01 Masse% Dimethylsulfoxid. Der Verbrauch an Präparat beträgt 10 kg/ha. Als Kontrolle dienen Pflanzen, behandelt mit einem Verdünner ohne Sterilisationsmittel.

Tabelle 8

| lfd. Nr. | Beispiel Nr. | Anzahl von Körnern der Ähre im Isolator | Prozentsatz der Sterilität | Anzahl von Körnern der Ähre bei freier Bestäubung | Prozentsatz des Ansetzens von Körnern bei freier Bestäubung |
|---|---|---|---|---|---|
| 1 | Kontrolle | 56,1 | 0,0 | 66,6 | 100,0 |
| 2 | Beispiel 32 | 0,0 | 100,0 | 58,2 | 87,4 |
| 3 | Beispiel 33 | 0,1 | 99,8 | 56,6 | 85,0 |
| 4 | Beispiel 34 | 0,0 | 100,0 | 60,1 | 90,2 |
| 5 | Beispiel 35 | 0,0 | 100,0 | 63,8 | 95,8 |
| 6 | Beispiel 36 | 0,0 | 100,0 | 60,5 | 90,8 |

Beispiele 37 bis 43

Pflanzen von diploidem Roggen Sorte Woschod 2 werden in der V. Periode der Organogenese (nach Kupermann) mit 1%iger wäßriger Emulsion von Bis(di-n-butyläther) der $\delta, \delta'$-Thiodivaleriansäure, 4-Krboxybutyldezylsulfid, Natriumsalz der $\delta, \delta'$-Thiodivaleriansäure, Bis(2-äthylhexyläther) der $\delta, \delta'$-Thiodivaleriansäure, $\delta, \delta'$-Sulfonyldivaleriansäure, Natriumsalz der $\delta, \delta'$-Sulfonyldivaleriansäure, Kaliumsalz der $\delta, \delta'$-Sulfonyldivaleriansäure behandelt. Die Emulsion enthält 0,1 Masse% Kalziumalkylbenzol mit 12 bis 14 Kohlenstoffatomen und 0,01 Masse% Tetrahydrofuran. Der Verbrauch an Präparat beträgt 6 kg/ha. Als Kontrolle dienen Pflanzen, behandelt mit einem Verdünner ohne Sterilisationsmittel.

Die Prüfergebnisse sind in der Tabelle 9 angegeben. Die ähnlichen Ergebnisse sind bei der Behandlung von diploidem Roggen Sorte Woschod 2 in der VI. Periode der Organogenese erhalten.

Tabelle 9

| lfd. Nr. | Beispiel Nr. | Anzahl von Körnern der Ähre im Isolator | Prozent-satz der Sterilität | Anzahl von Körnern der Ähre bei freier Bestäubung | Prozent-satz des Ansetzens von Körnern bei freier Bestäubung |
|---|---|---|---|---|---|
| 1 | Kontrolle | 49,8 | 0,0 | 54,6 | 100,0 |
| 2 | Beispiel 37 | 0,0 | 100,0 | 50,9 | 93,2 |
| 3 | Beispiel 38 | 0,0 | 100,0 | 50,5 | 92,5 |
| 4 | Beispiel 39 | 0,1 | 99,8 | 50,5 | 92,5 |
| 5 | Beispiel 40 | 0,0 | 100,0 | 52,9 | 96,9 |
| 6 | Beispiel 41 | 0,1 | 99,8 | 48,4 | 88,6 |
| 7 | Beispiel 42 | 0,1 | 99,8 | 47,4 | 86,8 |
| 8 | Beispiel 43 | 0,6 | 98,8 | 53,8 | 98,5 |

Beispiele 44 bis 50

Pflanzen von diploidem Roggen Sorte Gibrid 1861/79 werden in der V. und VI. Periode der Organogenese (nach Kupermann) mit 1%iger wäßriger Emulsion von Bis(di-n-butyläther) der $\delta, \delta'$-Thiodivaleriansäure, $\delta, \delta'$-Thiodivaleriansäure, Natriumsalz der $\delta, \delta'$-Thiodivaleriansäure, 4-Karboxybutyldezylsulfid, $\delta, \delta'$-Sulfonyldivaleriansäure, Kaliumsalz der $\delta, \delta'$-Sulfonyldivaleriansäure, Bis(2-äthylhexyläther) der $\delta, \delta'$-Thiodivaleriansäure behandelt. Die Emulsion enthält 0,1 Masse% Kalziumalkylbenzolsulfonat mit 12 bis 14 Kohlenstoffatomen und 0,01 Masse% Dimethylformamid. Der Verbrauch an Präparat beträgt 8 kg/ha. Als Kontrolle dienen Pflanzen, behandelt mit einem Verdünner ohne Sterilisationsmittel. Die Prüfergebnisse sind in der Tabelle 10 angegeben.

Tabelle 10

| lfd. Nr. | Beispiel Nr. | Anzahl von Körnern der Ähre im Isolator | Prozentsatz der Steri-lität | Anzahl von Körnern der Ähre bei freier Be-stäubung | Prozent-satz des Anset-zens von Körnern bei frei-er Be-stäubung |
|---|---|---|---|---|---|
| 1 | Kontrolle | 54,0 | 0,0 | 62,8 | 100,0 |
| 2 | Beispiel 44 | 0,0 | 100,0 | 60,0 | 95,5 |
| 3 | Beispiel 45 | 0,1 | 99,8 | 58,6 | 93,3 |
| 4 | Beispiel 46 | 0,1 | 99,8 | 57,1 | 90,9 |
| 5 | Beispiel 47 | 0,0 | 100,0 | 52,8 | 84,1 |
| 6 | Beispiel 48 | 0,0 | 100,0 | 54,7 | 87,1 |
| 7 | Beispiel 49 | 0,0 | 100,0 | 57,0 | 90,8 |
| 8 | Beispiel 50 | 0,0 | 100,0 | 53,2 | 84,7 |

Beispiele 51 bis 58

Pflanzen von tetraploidem Roggen Sorte Belta werden in der V. Periode der Organogenese (nach Kupermann) mit 1%iger wäßriger Emulsion von $\delta, \delta'$-Thiodivaleriansäure, Natriumsalz der $\delta, \delta'$-Thiodivaleriansäure, $\delta, \delta'$-Sulfonyl-valeriansäure, Natriumsalz der $\delta, \delta'$-Sulfonyldivaleri-ansäure, Kaliumsalz der $\delta, \delta'$-Sulfonyldivaleriansäure, Bis(di-n-butyläther) der $\delta, \delta'$-Thiodivaleriansäure, Bis(2-Äthylhexyläther) der $\delta, \delta'$-Thiodivaleriansäure, 4-Karboxybutyldezylsulfid behandelt. Die Emulsion enthält 0,1 Masse% Kalziumalkylbenzolsulfonat und 0,01 Masse% Dimethylsulfoxid. Der Verbrauch an Präparat beträgt 6 kg/ha. Als Kontrolle dienen Pflanzen, behandelt mit einem Verdünner ohne Sterilisationsmittel. Die Prüfergebnisse sind in der Tabelle 11 angegeben. Die ähnlichen Ergebnisse sind bei der Behandlung von tetraploidem Roggen Sorte Belta in der VI. Periode der Organogese erhalten.

Tabelle 11

| lfd. Nr. | Beispiel Nr. | Anzahl von Körnern der Ähre im Isolator | Prozent-satz der Sterili-tät | Anzahl von Körnern der Ähre bei freier Be-stäubung | Prozent-satz des Ansetzens von Körnern bei freier Be-stäubung |
|---|---|---|---|---|---|
| 1 | Kontrolle | 39,9 | 0,0 | 44,2 | 100,0 |
| 2 | Beispiel 51 | 0,3 | 99,3 | 32,9 | 74,4 |
| 3 | Beispiel 52 | 0,1 | 99,8 | 40,6 | 91,8 |
| 4 | Beispiel 53 | 0,0 | 100,0 | 39,0 | 88,2 |
| 5 | Beispiel 54 | 0,0 | 100,0 | 34,6 | 78,2 |
| 6 | Beispiel 55 | 0,0 | 100,0 | 34,0 | 76,9 |
| 7 | Beispiel 56 | 0,0 | 100,0 | 38,2 | 86,7 |
| 8 | Beispiel 57 | 0,0 | 100,0 | 42,7 | 96,6 |
| 9 | Beispiel 58 | 0,0 | 100,0 | 40,3 | 91,2 |

Beispiele 59 bis 61

Pflanzen von Tritikale PRAG 109 werden in der V. Periode der Organogenese (nach Kupermann) mit 1,5%iger wäßriger Emulsion von Bis(di-n-butyläther) der $\delta$, $\delta'$ -Thiodivaleriansäure, 4-Karboxybutyldezylsulfid, Bis(2-Äthylhexyläther) der $\delta$, $\delta'$ -Thiodivaleriansäure behandelt. Die Emulsion enthält 0,1 Masse% Kalziumalkylbenzolsulfonat mit 12 bis 14 Kohlenstoffatomen und 0,01 Masse% Tetrahydrofuran. Der Verbrauch an Präparat beträgt 15 kg/ha. Als Kontrolle dienen Pflanzen, behandelt mit einem Verdünner ohne Sterilisationsmittel. Die Prüfergebnisse sind in der Tabelle 12 angegeben. Die ähnlichen Ergebnisse sind bei der Behandlung von Tritikale PRAG 109 in der VI. Periode der Organogenese erhalten.

Tabelle 12

| lfd. Nr. | Beispiel Nr. | Anzahl von Körnern der Ähre im Isolator | Prozentsatz der Sterilität | Anzahl von Körnern der Ähre bei freier Bestäubung | Prozentsatz des Ansetzens von Körnern bei freier Bestäubung |
|---|---|---|---|---|---|
| 1 | Kontrolle | 41,3 | 0,0 | 45,8 | 100,0 |
| 2 | Beispiel 59 | 0,3 | 99,3 | 40,1 | 87,6 |
| 3 | Beispiel 60 | 0,0 | 100,0 | 42,3 | 92,4 |
| 4 | Beispiel 61 | 0,0 | 100,0 | 40,8 | 89,1 |

Beispiele 62 bis 64

Pflanzen von Tritikale der Sorte Amphidiploid 206 werden in der V. Periode der Organogenese (nach Kupermann) mit 1,5%iger wäßriger Emulsion von Natriumsalz der $\delta$, $\delta'$-Thiodivaleriansäure, $\delta$, $\delta'$-Sulfonyldivaleriansäure Kaliumsalz der $\delta$, $\delta'$-Sulfonyldivaleriansäure behandelt. Die Emulsion enthält 0,1 Masse% Kalziumalkylbenzolsulfonat mit 12 bis 14 Kohlenstoffatomen und 0,01 Masse% Dodezylsulfat. Der Verbrauch an Präparat beträgt 9 kg/ha. Als Kontrolle dienen Pflanzen, behandelt mit einem Verdünner ohne Sterilisationsmittel. Die Prüfergebnisse sind in der Tabelle 13 angegeben. Die ähnlichen Ergebnisse sind bei der Behandlung von Tritikale Sorte Amphidiploid 206 in der VI. Periode der Organogenese erhalten.

Tabelle 13

| lfd. Nr. | Beispiel Nr. | Anzahl von Körnern der Ähre im Isolator | Prozentsatz der Sterilität | Anzahl von Körnern der Ähre bei freier Bestäubung | Prozentsatz des Ansetzens von Körnern bei freier Bestäubung |
|---|---|---|---|---|---|
| 1 | Kontrolle | 32,7 | 0,0 | 36,6 | 100,0 |
| 2 | Beispiel 62 | 0,9 | 97,2 | 32,5 | 88,8 |
| 3 | Beispiel 63 | 0,1 | 99,7 | 30,1 | 82,2 |
| 4 | Beispiel 64 | 0,0 | 100,0 | 29,8 | 81,4 |

Beispiele 65 bis 67

Pflanzen der Hirse Sorte Mironowskoe 94 werden in der V. Periode der Organogenese (nach Kupermann) mit 1%iger wäßriger Emulsion von 4-Karboxybutyldezylsulfid, $\delta, \delta'$ -Sulfonyldivaleriansäure, Bis(2-äthylhexyläther) der $\delta, \delta'$ -Thiodivaleriansäure behandelt. Die Emulsion enthält 0,1 Masse% Kalziumalkylbenzolsulfonat mit 12 bis 14 Kohlenstoffatomen und 0,01 Masse% Dodezylsulfat. Der Verbrauch an Präparat beträgt 10 kg/ha. Als Kontrolle dienen Pflanzen, behandelt mit einem Verdünner ohne Sterilisationsmittel. Die Prüfergebnisse sind in der Tabelle 14 angegeben. Die ähnlichen Ergebnisse sind bei der Behandlung der Hirse Sorte Mironowskoe 94 in der VI. Periode der Organogenese erhalten.

Tabelle 14

| lfd. Nr. | Beispiel Nr. | Anzahl von Körnern der Rispe im Isolator | Prozentsatz der Sterilität | Anzahl von Körnern der Rispe bei freier Bestäubung | Prozentsatz des Ansetzens von Körnern bei freier Bestäubung |
|---|---|---|---|---|---|
| 1 | Kontrolle | 324,5 | 0,0 | 366,4 | 100,0 |
| 2 | Beispiel 65 | 0,0 | 100,0 | 323,7 | 88,3 |
| 3 | Beispiel 66 | 0,0 | 100,0 | 336,8 | 91,9 |
| 4 | Beispiel 67 | 0,0 | 100,0 | 312,8 | 85,3 |

Beispiele 68 bis 72

Pflanzen der Hirse Sorte K9009, Poltawskaja oblast (Poltawaer Gebiet) werden in der V.Periode der Organogenese (nach Kupermann) mit 1%iger wäßriger Emulsion von $\delta, \delta'$ - Thiodivaleriansäure, Natriumsalz der $\delta, \delta'$- -Thiodivaleriansäure, $\delta, \delta'$ -Sulfonyldivaleriansäure, Bis(2-äthylglykoläther) der $\delta, \delta'$ -Thiodivaleriansäure, 4-Karboxybutyldezylsulfid behandelt. Die Emulsion enthält 0,1 Masse% Kalziumalkylbenzolsulfonat mit 12 bis 14 Kohlenstoffatomen und 0,01 Masse% Tetrahydrofuran, Der Verbrauch an Präparat beträgt 6 kg/ha. Als Kontrolle dienen Pflanzen, behandelt mit einem Verdünner ohne Sterilisationsmittel. Die Prüfergebnisse sind in der Tabelle 15 angegeben. Die ähnlichen Ergebnisse sind bei der Behandlung der Hirse Sorte K9009, Poltawskaja oblast (Poltawaer Gebiet) in der VI. Periode der Organogenese erhalten.

Tabelle 15

| lfd. Nr. | Beispiel Nr. | Anzahl von Körnern der Rispe im Isolator | Prozentsatz der Sterilität | Anzahl von Körnern der Rispe bei freier Bestäubung | Prozentsatz des Ansetzens von Körnern bei freier Bestäubung |
|---|---|---|---|---|---|
| 1 | Kontrolle | 120,8 | 0,0 | 129,4 | 100,0 |
| 2 | Beispiel 68 | 0,0 | 100,0 | 101,4 | 78,1 |
| 3 | Beispiel 69 | 0,0 | 100,0 | 105,1 | 81,2 |
| 4 | Beispiel 70 | 0,0 | 100,0 | 77,1 | 60,0 |
| 5 | Beispiel 71 | 0,0 | 100,0 | 112,0 | 86,6 |
| 6 | Beispiel 72 | 0,0 | 100,0 | 100,7 | 77,8 |

Beispiele 73 und 74

Pflanzen der Hirse Sorte K-9693 Kormowoe 1 werden in der V. Periode der Organogenese (nach Kupermann) mit 1%iger wäßriger Emulsion von Bis(di-n-butyläther) der $\delta, \delta'$-Thiodivaleriansäure, Natriumsalz der $\delta, \delta'$-Sulfonyldivaleriansäure behandelt. Der Verbrauch an Präparat beträgt 6 kg/ha. Als Kontrolle dienen Pflanzen, behandelt mit einem Verdünner ohne Sterilisationsmittel. Die Prüfergebnisse sind in der Tabelle 16 angegeben. Die ähnlichen Ergebnisse sind bei der Behandlung der Hirse Sorte K-9693 Kormowoe 1 in der VI. Periode der Organogenese erhalten.

Tabelle 16

| lfd. Nr. | Beispiel Nr. | Anzahl von Körnern der Rispe im Isolator | Prozentsatz der Sterilität | Anzahl von Körnern der Rispe bei freier Bestäubung | Prozentsatz des Ansetzens von Körnern bei freier Bestäubung |
|---|---|---|---|---|---|
| 1 | Kontrolle | 125,0 | 0,0 | 146,7 | 100,0 |
| 2 | Beispiel 73 | 0,0 | 100,0 | 122,1 | 83,2 |
| 3 | Beispiel 74 | 0,0 | 100,0 | 103,5 | 70,5 |

Beispiele 75 bis 88

Man behandelt die Sonnenblume Sorte ВНИИМК 8931 (WNIIMK 8931) in der V. Periode der Organogenese mit 0,5%iger wäßriger Emulsion von 4-Karboxybutyldezylsulfid, mit 1%iger Emulsion der $\delta$, $\delta'$-Sulfonyldivaleriansäure, 2%iger Emulsion von Bis(2-äthylhexyläther) der $\delta$, $\delta'$-Thiodivaleriansäure; die Sonnenblume der Linie BK-373 (WK-373) mit 2%iger Emulsion von Bis(2-äthylhexyläther) der $\delta$, $\delta'$-Thiodivaleriansäure, mit 0,2%iger Emulsion von Bis(di-n-butyläther) der $\delta$, $\delta'$-Thiodivaleriansäure; die Sonnenblume der Linie BK-364 (WK-365) mit 2%iger wäßriger Emulsion von Bis(2-äthylhexyläther) der $\delta$, $\delta'$-Thiodivaleriansäure, mit 0,2%iger Emulsion von Bis(di-n-butyläther) der $\delta$, $\delta'$-Thiodivaleriansäure. Die Emulsion enthält 0,1 Masse% Kalziumalkylbenzolsulfonat mit 12 bis 14 Kohlenstoffatomen und 0,01 Masse% Dimethylsulfoxid. Als Kontrolle dienen Pflanzen, behandelt mit einem Verdünner ohne Sterilisationsmittel. Die Prüfergebnisse sind in der Tabelle 17 angegeben. Die ähnlichen Ergebnisse sind bei der Behandlung der Sonnenblume der angegebenen Sorten in der VI. Periode der Organogenese erhalten.

Tabelle 17

| lfd. Nr. | Beispiel Nr. | Prozentsatz der Sterilität | Prozentsatz des Ansetzens von Achänen bei freier Bestäubung | Masse von 1000 Achänen in g | Ölgehalt in % |
|---|---|---|---|---|---|
| 1 | 2 | 3 | 4 | 5 | 6 |
| Sorte ВНИИМК 8931 (WNIIMK 8931) | | | | | |
| 1 | Kontrolle | 0,5 | 85,7 | 83,0 | 54,7 |
| 2 | Beispiel 75 | 100,0 | 82,9 | 78,0 | 52,5 |
| 3 | Beispiel 76 | 100,0 | 71,6 | 85,5 | 53,6 |
| 4 | Beispiel 77 | 100,0 | 82,1 | 91,9 | 53,9 |
| Linie BK-373 (WK-373) | | | | | |
| 5 | Kontrolle | 0,7 | 67,9 | 58,1 | 47,6 |
| 6 | Beispiel 78 | 100,0 | 60,7 | 54,5 | 48,1 |
| 7 | Beispiel 79 | 100,0 | 62,7 | 57,2 | 49,2 |

Fortsetzung der Tabelle 17

| 1 | 2 | 3 | 4 | 5 | 6 |
|---|---|---|---|---|---|
| | | Linie BK-364 (WK-364) | | | |
| 8 | Kontrolle | 0,5 | 70,3 | 87,3 | 45,6 |
| 9 | Beispiel 80 | 100,0 | 67,9 | 87,0 | 45,5 |
| 10 | Beispiel 81 | 100,0 | 59,3 | 87,1 | 44,5 |

Fortsetzung der Tabelle 17

| lfd. Nr. | Beispiel Nr. | Keimfähigkeit in % | Verbrauch je ha in kg/600 l |
|---|---|---|---|
| | | Sorte ВНИИМК 8931 (WNIIMK 8931) | |
| 1 | Kontrolle | 100,0 | – |
| 2 | Beispiel 82 | 100,0 | 3,0 |
| 3 | Beispiel 83 | 100,0 | 6,0 |
| 4 | Beispiel 84 | 100,0 | 12,0 |
| | | Linie BK-373 (WK-373) | |
| 5 | Kontrolle | 100,0 | – |
| 6 | Beispiel 85 | 100,0 | 12,0 |
| 7 | Beispiel 86 | 100,0 | 1,2 |
| | | Linie BK-364 (WK-364) | |
| 8 | Kontrolle | 100,0 | – |
| 9 | Beispiel 87 | 100,0 | 12,0 |
| 10 | Beispiel 88 | 100,0 | 1,2 |

## Gewerbliche Anwendbarkeit

Das angemeldete Verfahren findet in der Selektion und in Samenbau zur Gewinnung hochproduktiver Sorten und Hybriden von landwirtschaftlichen Kulturen seine Anwendung.

PATENTANSPRÜCHE

1. Verfahren zur Sterilisation der Staubbeutel von Pflanzen durch Behandlung derselben mit einem Sterilisationsmittel in Verbindung mit einem Verdünner in der fünften und/oder sechsten Periode der Organogenese, dadurch gekennzeichnet, daß als Sterilisationsmittel Thiokarbonsäuren und/oder ihre Derivate der allgemeinen Formel RXR' dienen, worin X für $S$, $SO_2$, R für $(CH_2)_4CO_2H$; $(CH_2)_4CO_2Na$; $(CH_2)_4CO_2K$; $(CH_2)_4CO_2C_4H_9$; $(CH_2)_4CO_2C_8H_{17}$; $C_{10}H_{21}$; R' für $(CH_2)_4CO_2H$; $(CH_2)_4CO_2Na$; $(CH_2)_4CO_2K$; $(CH_2)_4CO_2C_4H_9$; $(CH_2)_4CO_2C_8H_{17}$ stehen.

2. Verfahren nach Anspruch 1, dadurch gekennzeichnet, daß die angegebenen Thiokarbonsäuren und/oder ihre Derivate in Verbindung mit einem Verdünner, und zwar, Wasser, als 0,1- bis 2%ige wäßrige Emulsion verwendet werden.

3. Verfahren nach einem der Ansprüche 1 bis 2, dadurch gekennzeichnet, daß als Pflanzen, die mit dem Sterilisationsmittel zu behandeln sind, Graspflanzen und Sonnenblume dienen.

4. Verfahren nach einem der Ansprüche 1 bis 3, dadurch gekennzeichnet, daß zwecks Erzielung eines hohen Sterilisationsgrades von Staubbeuteln unter ungünstigen Klimabedingungen eine wiederholte Behandlung von Pflanzen mit dem Sterilisationsmittel in der fünften und/oder sechsten Periode der Organogenese erfolgt.

# VERFAHREN ZUR STERILISATION DER STAUBBEUTEL VON PFLANZEN

## ZUSAMMENFASSUNG

Das Verfahren zur Sterilisation der Staubbeutel von Pflanzen sieht die Behandlung von Pflanzen mit einem Sterilisationsmittel in Verbindung mit einem Verdünner in der fünften und/oder sechsten Periode der Organogenese vor. Als Sterilisationsmittel verwendet man erfindungsgemäß Thiokarbonsäuren und/oder ihre Derivate der allgemeinen Formel RXR', worin X für $S$, $SO_2$; R für $(CH_2)_4CO_2H$; $(CH_2)_4CO_2Na$; $(CH_2)_4CO_2K$; $(CH_2)_4CO_2C_4H_9$; $(CH_2)_4CO_2C_8H_{17}$; $C_{10}H_{21}$; R' für $(CH_2)_4CO_2H$; $(CH_2)_4CO_2Na$; $(CH_2)_4CO_2K$; $(CH_2)_4CO_2C_4H_9$; $(CH_2)_4CO_2C_8H_{17}$ stehen.

# INTERNATIONAL SEARCH REPORT

International Application No PCT/SU 88/00060

## I. CLASSIFICATION OF SUBJECT MATTER (if several classification symbols apply, indicate all) *

According to International Patent Classification (IPC) or to both National Classification and IPC

IPC$^4$  A01N 41/12,41/10,37/36

## II. FIELDS SEARCHED

### Minimum Documentation Searched 7

| Classification System | Classification Symbols |
|---|---|
| IPC$^4$ | A01N 37/36, 41/10, 41/12;A01H 1/04 |

### Documentation Searched other than Minimum Documentation to the Extent that such Documents are Included in the Fields Searched 8

## III. DOCUMENTS CONSIDERED TO BE RELEVANT *

| Category * | Citation of Document, 11 with indication, where appropriate, of the relevant passages 12 | Relevant to Claim No. 13 |
|---|---|---|
| A | SU, A1, 640712 (Vsesojuzny ordena Lenina i ordena Druzhby narodov nauchno-issledovatelsky institut rastenievodstva im N.N. Vavilova et al.) 09 January 1979 (09.01.79) | 1-3 |
| A | US, A, 4459152 (Ciba-Geigy Corporation) 10 July 1984 (10.07.84) see columns 18,19 | 1-4 |
| A | EP, A1, 0037133 (SHELL INTERNATIONALE RESEARCH MAATSHAPPIJ B.V.) 07 October 1981 (07.10.81) see columns 21,22 | 1-4 |
| A | L. Dzh. Nikell "Regulyatory rosta rasteny Primenenie v selskom khozyaistve" 1984, Kolos, (Moscow), pages 30-31 (cited in the description) | 1 |
| A | Plant Breeding Reviews, edited by Jules Janick Purdue University, vol. 3, 1985 AVI Publishing Company, Inc.,(Wesport Connecicut, USA) see pages 169-186 | 1 |

* Special categories of cited documents: 10
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art.
"&" document member of the same patent family

## IV. CERTIFICATION

| Date of the Actual Completion of the International Search | Date of Mailing of this International Search Report |
|---|---|
| 10 October 1988 (10.10.88) | 16 December 1988 (16.12.88) |
| International Searching Authority | Signature of Authorized Officer |
| ISA/SU | |

Form PCT/ISA/210 (second sheet) (January 1985)

| Category* | Citation of Document, with indication, where appropriate, of the relevant passages | Relevant to Claim No |
|---|---|---|
| A | FR, A1, 2389324 (IMPERIAL CHEMICAL INDUSTRIES LIMITED) 01 December 1978 (01.12.78) see pages 1-3,13-17 | 1 |